(19)

**Europäisches Patentamt**
**European Patent Office**
**Office européen des brevets**

(11) **EP 3 689 243 A1**

(12) **EUROPEAN PATENT APPLICATION**

(43) Date of publication:
**05.08.2020 Bulletin 2020/32**

(51) Int Cl.:
**A61B 6/10** *(2006.01)*  **A61B 6/00** *(2006.01)*
**A61B 6/03** *(2006.01)*

(21) Application number: **20154978.9**

(22) Date of filing: **31.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.02.2019 KR 20190013463**

(71) Applicant: **Samsung Electronics Co., Ltd.**
**Gyeonggi-do 16677 (KR)**

(72) Inventors:
• **I, Gitae**
  **16677 Gyeonggi-do (KR)**
• **CHO, Minkook**
  **16677 Gyeonggi-do (KR)**
• **HSIEH, Jongjyh**
  **16677 Gyeonggi-do (KR)**
• **KIM, Daehwan**
  **16677 Gyeonggi-do (KR)**

(74) Representative: **Arnold & Siedsma**
**Bezuidenhoutseweg 57**
**2594 AC The Hague (NL)**

(54) **MEDICAL IMAGING APPARATUS AND CONTROL METHOD OF THE SAME**

(57)     Disclosed herein is a medical imaging apparatus and a control method of the same. The medical imaging apparatus including a rotatable gantry in which an X-ray generator configured to generate X-rays and emit the X-rays to an object and an X-ray detector configured to detect the X-rays emitted from the X-ray generator are arranged to face each other, the medical imaging apparatus includes a mover configured to move the medical imaging apparatus, a sensor configured to measure position data according to the movement of the gantry; generate image data based on the detected X-rays, correct the image data based on the measured position data, and generate an X-ray computed tomography (CT) image based on the corrected image data.

**FIG. 2**

EP 3 689 243 A1

**Description**

CROSS-REFERENCE TO RELATED APPLICATION(S)

This application is based on and claims priority under 35 U.S.C. §119 to Korean Patent Application No. 10-2019-0013463, filed on February 01, 2019, in the Korean Intellectual Property Office, the disclosure of which is incorporated by reference herein in its entirety.

BACKGROUND

**1. Field of the Invention**

The disclosure relates to a medical imaging apparatus, and more particularly, to a mobile computed tomography imaging apparatus configured to generate an X-ray computed tomography image and a control method of the same.

2. **Description of Related Art**

A medical imaging apparatus is a device for acquiring an image of an internal structure of an object. The medical imaging apparatus is a non-invasive inspection apparatus, which images and processes structural details, internal tissues, and fluid flows in the body and shows them to the user. A user such as a doctor may diagnose a medical condition and a disease of a patient by using a medical image output from the medical imaging apparatus.
A computed tomography (CT) apparatus is a typical example for an apparatus for imaging an object by emitting X-rays to a patient.
Such a computed tomography (CT) imaging apparatus (hereinafter referred to as a CT imaging apparatus) may provide an image of an object, and further represent an internal structure (for example, an organ such as the kidney or the lung) without superimposing images of the object in comparison with a general X-ray apparatus. Due to the advantages, the CT imaging apparatus has been widely used for precise diagnosis of disease. Hereinafter a medical image acquired by the CT imaging apparatus is called a computed tomography (CT) image. As for the CT imaging apparatus in the conventional manner, CT imaging is performed as a table on which an object is placed and is inserted into the CT imaging apparatus. The CT imaging apparatus reconstructs a CT image of the object by using data acquired through the CT imaging.
Recently, in order to improve convenience and usability of the CT imaging apparatus, a mobile CT imaging apparatus capable of being moved to an object has been developed. The mobile CT imaging apparatus is directly moved to an object having difficulty with movement and performs the CT imaging by scanning the object instead of a table on which the object is placed.
However, the mobile CT imaging apparatus is likely to perform the CT imaging in a space having an uneven floor on which an object is placed, which may cause difficulties in the CT imaging that requires a precise scan.

SUMMARY OF THE INVENTION

Therefore, it is an aspect of the disclosure to provide a mobile computed tomography (CT) imaging apparatus, by applying a vibration, which may occur in computed tomography imaging of the mobile CT imaging apparatus, to an image reconstruction, capable of improving the usability of the mobile CT imaging apparatus, providing an improved CT image, and preventing misdiagnosis caused by an incorrect CT image, and a control method of the same.
Additional aspects of the disclosure will be set forth in part in the description which follows and, in part, will be obvious from the description, or may be learned by practice of the disclosure.
In accordance with an aspect of the disclosure, a medical imaging apparatus including a rotatable gantry in which an X-ray generator configured to generate X-rays and emit the X-rays to an object and an X-ray detector configured to detect the X-rays emitted from the X-ray generator are arranged to face each other, the medical imaging apparatus includes a mover configured to move the medical imaging apparatus, a sensor configured to measure position data according to the movement of the gantry; and a controller configured to generate image data based on the detected X-rays, correct the image data based on the measured position data, and generate an X-ray computed tomography (CT) image based on the corrected image data.
The position data may include at least one of a movement value according to the movement of the gantry, a rotation angle according to the movement of the gantry, or a vibration value according to the rotation of the gantry, and the controller may correct the image data based on a plurality of pieces of position data.
The controller may generate the image data based on the detected X-rays and the position data.
The controller may match position data that is detected by the sensor in each section in which the gantry is moved, with image data that is generated according to the movement of the gantry, and the controller may correct the image data based on the matched position data.

The sensor may include at least one of an ultrasonic sensor, a laser sensor, an acceleration sensor, or a gyro sensor.

The sensor may include a plurality of sensors configured to measure a distance to a table on which the object is placed.

The medical imaging apparatus may further include a storage configured to store first position data including distances between the table and the plurality of sensors. The controller may compare second position data detected according to the movement of the gantry with the first position data, and correct the image data based on a comparison result.

The sensor may detect an obstacle positioned in a direction in which the gantry is moved, and the controller may control the mover based on a predetermined expected path and the detected obstacle.

The sensor may include a beam projector configured to emit laser beams to the direction in which the gantry is moved, and a camera configured to image an obstacle distinguished by the laser beam, and the controller may detect an obstacle based on the image imaged by the camera.

The controller may control the mover to allow the gantry to move straight on the detected obstacle.

The controller may control the beam projector to emit the laser beam based on a length of the table on which the object is placed.

The mover may include a first wheel, a first motor configured to provide a driving force to the first wheel, a second wheel configured to move the gantry during the X-rays are emitted, and a second motor configured to provide a driving force to the second wheel, and the controller may control the second motor to regulate a rotation speed of the second motor based on the detected obstacle.

In accordance with another aspect of the disclosure, a control method of a medical imaging apparatus including a rotatable gantry in which an X-ray generator configured to generate X-rays and emit the X-rays to an object and an X-ray detector configured to detect the X-rays emitted from the X-ray generator are arranged to face each other, the control method includes moving the medical imaging apparatus, generating image data based on the detected X-rays, measuring position data according to movement of the gantry, correcting the image data based on the measured position data, and generating an X-ray computed tomography (CT) image based on the corrected image data.

In accordance with another aspect of the disclosure, a control method of a medical imaging apparatus including a rotatable gantry in which an X-ray generator configured to generate X-rays and emit the X-rays to an object and an X-ray detector configured to detect the X-rays emitted from the X-ray generator are arranged to face each other, the control method includes moving the medical imaging apparatus, measuring position data according to movement of the gantry, generating the image data based on the measured position data, and generating an X-ray computed tomography (CT) image based on the generated image data.

The position data may include at least one of a movement value according to the movement of the gantry, a rotation angle according to the movement of the gantry, or a vibration value according to the rotation of the gantry, and the correction of the image data may include correcting the image data based on a plurality of pieces of position data.

The generation of the image data may include generating the image data based on the detected X-rays and the position data.

The correction of the image data may include matching the position data, which is detected at a time in which the gantry is moved, with image data generated according to the movement of the gantry, and correcting the image data based on the matched position data.

The correction of the image data may include correcting the image data based on position data about a distance to a table on which the object is placed.

The movement of the gantry may include moving the gantry based on a rotational force provided to a second wheel after inserting a first wheel into the inside of a main body.

The control method may further include detecting an obstacle located in a direction in which the gantry is moved, and controlling the gantry to move straight based on a predetermined expected path and the detected obstacle.

The controlling may include increasing a rotation speed of a motor configured to provide a driving force to a wheel moved on a path on which the obstacle is detected.

BRIEF DESCRIPTION OF THE DRAWINGS

These and/or other aspects of the disclosure will become apparent and more readily appreciated from the following description of embodiments, taken in conjunction with the accompanying drawings of which:

FIG. 1 is a schematic view of a mobile computed tomography (CT) imaging apparatus according to an embodiment of the disclosure;

FIG. 2 is a diagram illustrating each configuration of the mobile CT imaging apparatus;

FIG. 3 is a diagram illustrating a configuration of a communication circuitry;

FIG. 4A is a view illustrating a mover of the mobile CT imaging apparatus;

FIG. 4B is a view illustrating the mover of the mobile CT imaging apparatus;

FIG. 4C is a view illustrating the mover of the mobile CT imaging apparatus;

FIG. 5 is a view of a sensor according an embodiment of the disclosure;

FIG. 6 is a flowchart illustrating a control method of a medical imaging apparatus according to an embodiment of the disclosure;

FIG. 7 is a view illustrating an example of receiving position data from the sensor;

FIG. 8 is a view illustrating a method of correcting image data based on the position data;

FIG. 9 is a view illustrating an example of the position data;

FIG. 10 is a flowchart illustrating a control method of a mover according to another embodiment of the disclosure;

FIG. 11 is a view illustrating an example of identifying an obstacle;

FIG. 12 is a view illustrating an example of identifying the obstacle;

FIG. 13A is a view illustrating an example of controlling the mover based on an obstacle identified;

FIG. 13B is a view illustrating an example of controlling the mover based on the obstacle identified; and

FIG. 13C is a view illustrating an example of controlling the mover based on the obstacle identified.

## DETAILED DESCRIPTION

In the following description, like reference numerals refer to like elements throughout the specification. Well-known functions or constructions are not described in detail since they would obscure the one or more exemplar embodiments with unnecessary detail. Terms such as "unit", "module", "member", and "block" may be embodied as hardware or software. According to embodiments, a plurality of "unit", "module", "member", and "block" may be implemented as a single component or a single "unit", "module", "member", and "block" may include a plurality of components.

It will be understood that when an element is referred to as being "connected" to another element, it can be directly or indirectly connected to the other element, wherein the indirect connection includes "connection via a wireless communication network".

Also, when a part "includes" or "comprises" an element, unless there is a particular description contrary thereto, the part may further include other elements, not excluding the other elements.

Throughout the description, when a member is "on" another member, this includes not only when the member is in contact with the other member, but also when there is another member between the two members.

It will be understood that, although the terms first, second, third, etc., may be used herein to describe various elements, but is should not be limited by these terms. These terms are only used to distinguish one element from another element.

As used herein, the singular forms "a," "an" and "the" are intended to include the plural forms as well, unless the context clearly indicates otherwise.

An identification code is used for the convenience of the description but is not intended to illustrate the order of each step. Each step may be implemented in the order different from the illustrated order unless the context clearly indicates otherwise.

Throughout the description, an "image" may mean multi-dimensional data formed of discrete image elements, e.g., pixels in a two-dimensional (2D) image and voxels in a three-dimensional (3D) image. For example, the image may include a medical image of an object which is imaged by a computed tomography (CT) imaging apparatus.

Throughout the description, a "CT image" or a "tomography image" may mean an image generated by synthesizing a plurality of X-ray images that are obtained by imaging an object while a CT imaging apparatus rotates around at least one axis with respect to the object.

Throughout the description, an "object" may be a human, an animal, or a portion of a human or animal. For example, the object may include at least one of an organ (e.g., the liver, heart, womb, brain, breast, or abdomen), or a blood vessel. Also, the object may be a phantom. The phantom means a material having a density, an effective atomic number, and a volume that are approximately the same as those of an organism. For example, the phantom may be a spherical phantom having properties similar to the physical body. In addition, the phantom may include an image quality evaluation phantom that may be used for evaluating the image quality of an image, and a calibration phantom used for estimating point spread function (PSF).

Throughout the description, a "user" may be, but is not limited to, a medical expert including a medical doctor, a nurse, a medical laboratory technologist, a medial image expert, or a technician who repairs a medical apparatus.

The medical imaging apparatus described below may include all tomography apparatuses such as a computed tomography (CT) apparatus, an optical coherence tomography (OCT) apparatus, or a positron emission tomography (PET)-CT apparatus.

The CT imaging apparatus may obtain a plurality of pieces of image data with a thickness not more than 2 mm several hundred times per second and then may process the plurality of pieces of image data, so that the CT imaging apparatus may provide a relatively accurate cross-sectional image of the object. According to the conventional manner, only a horizontal cross-sectional image of the object can be obtained, but this issue has been overcome due to various image reconstruction methods. Examples of 3D image reconstruction methods are as below:

- Shade surface display (SSD): an initial 3D imaging method of displaying only voxels having a predetermined Hounsfield Units (HU) value.
- Maximum intensity projection (MIP)/minimum intensity projection (MinIP): a 3D imaging method of displaying only voxels having the greatest or smallest HU value from among voxels that construct an image.
- Volume rendering (VR): an imaging method capable of adjusting a color and transmittance of voxels that constitutes an image, according to areas of interest.
- Virtual endoscopy: a method that allows endoscopy observation in a 3D image that is reconstructed by using the VR method or the SSD method.
- Multi-planar reformation (MPR): a method of reconstructing an image into a different cross-sectional image. A user may reconstruct an image in any desired direction.
- Editing: a method of editing adjacent voxels so as to allow a user to easily observe an area of interest in volume rendering.
- Voxel of interest (VOI): a method of displaying only a selected area.

Hereinafter a mobile computed tomography (CT) imaging apparatus will be described as an example of a medical imaging apparatus.

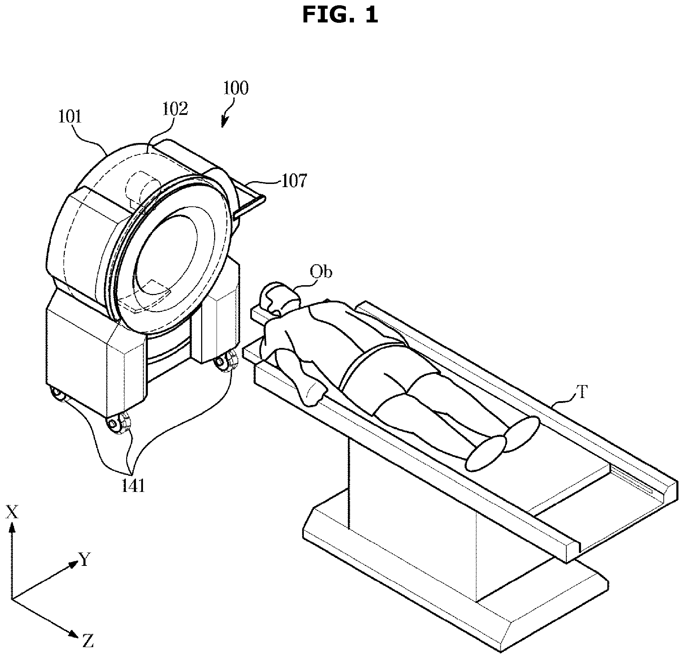

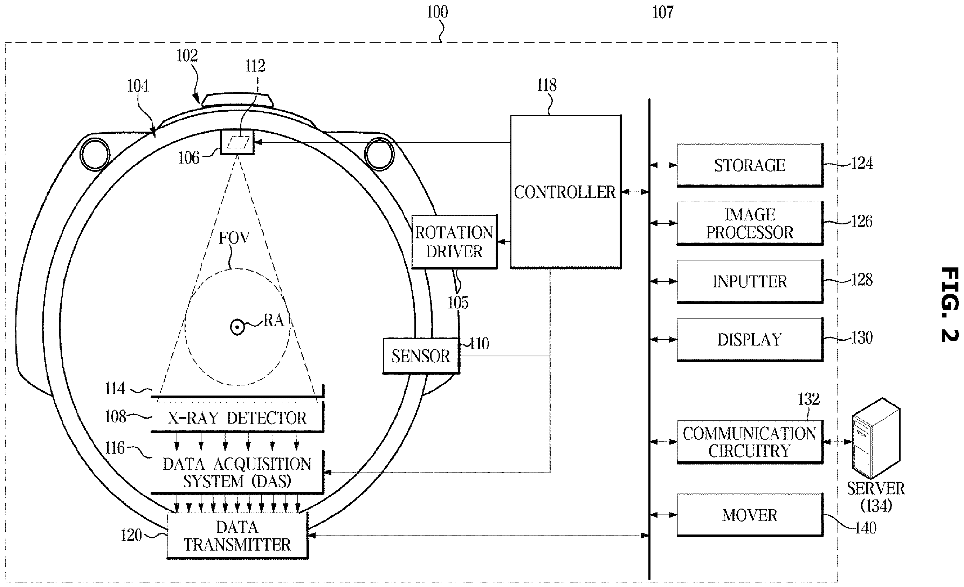

FIG. 1 is a schematic view of a disclosed mobile computed tomography (CT) imaging apparatus, and FIG. 2 is a diagram illustrating each configuration of the mobile CT imaging apparatus. In order to avoid duplicates, the description thereof will be described together.

Referring to FIG. 1, a mobile computed tomography (CT) imaging apparatus 100 may include a main body 101 including a gantry 102, a handle 107, and a wheel 141.

The mobile CT imaging apparatus 100 may be moved through the wheel 141 and may be moved to the Y axis side or the Z axis side by a mover 140 configured to provide a driving force to the wheel 141 as well as by a force, which is provided from a user through the handle 107.

Meanwhile, the disclosed mobile CT imaging apparatus 100 may perform computed tomography (CT) imaging while being moved in the Z-axis direction with respect to the table T on which the object Ob is placed. Detailed description thereof will be described later with reference to other drawings.

Referring to FIG. 2, the gantry 102 may include a rotating frame 104, an X-ray generator 106, an X-ray detector 108, a rotation driver 105, a data acquisition system (DAS) 116, and a data transmitter 120.

The gantry 102 may include the rotating frame 104 having a loop shape capable of rotating with respect to a predetermined rotation axis RA. Further, the rotating frame 104 may have a disc shape.

The rotating frame 104 may include the X-ray generator 106 and the X-ray detector 108 that are arranged to face each other so as to have predetermined fields of view (FOV). The rotating frame 104 may also include an anti-scatter grid 114. The anti-scatter grid 114 may be arranged between the X-ray generator 106 and the X-ray detector 108.

X-ray radiation that reaches a detector (or a photosensitive film) includes not only attenuated primary radiation that forms a valuable image but also scattered radiation that deteriorates the quality of an image. In order to transmit most of the primary radiation and to attenuate the scattered radiation, the anti-scatter grid 114 may be positioned between a patient and the detector (or the photosensitive film).

For example, the anti-scatter grid 114 may be formed by alternately stacking strips of lead foil and an interspace material such as a solid polymer material, solid polymer, or a fiber composite material. However, formation of the anti-scatter grid 114 is not limited thereto.

The rotating frame 104 may receive a driving signal from the rotation driver 105 and may rotate the X-ray generator 106 and the X-ray detector 108 at a predetermined rotation speed. The rotating frame 104 may receive the driving signal and power from the rotation driver 105 while the rotating frame 104 contacts the rotation driver 105 via a slip ring (not shown). Further, the rotating frame 104 may receive the driving signal and power from the rotation driver 105 via wireless communication.

The X-ray generator 106 may receive a voltage and current from a power distribution unit (PDU) (not shown) via a slip ring (not shown) and then a high voltage generator (not shown), and may generate and emit an X-ray. When the high voltage generator applies a predetermined voltage (hereinafter, referred to as a tube voltage) to the X-ray generator 106, the X-ray generator 106 may generate X-rays having a plurality of energy spectra that correspond to the tube voltage.

The X-ray generated by the X-ray generator 106 may be emitted in a predetermined form or in a predetermined area due to a collimator 112.

The X-ray detector 108 may be arranged to face the X-ray generator 106. The X-ray detector 108 may include a plurality of X-ray detector elements. A single X-ray detector element may establish one channel but is not limited thereto.

The X-ray detector 108 may detect the X-ray that is generated by the X-ray generator 106 and that is transmitted through the object 10, and may generate an electrical signal corresponding to intensity of the detected X-ray.

The X-ray detector 108 may include an indirect-type X-ray detector for detecting radiation after converting the radiation into light, and a direct-type X-ray detector for detecting radiation after directly converting the radiation into electric charges. The indirect-type X-ray detector may use a scintillator. Further, the direct-type X-ray detector may use a photon counting detector.

The data acquisition system (DAS) 116 may be connected to the X-ray detector 108. Electrical signals generated by the X-ray detector 108 may be collected by the DAS 116. Electrical signals generated by the X-ray detector 108 may be collected by wire or wirelessly by the DAS 116.

Further, the electrical signals generated by the X-ray detector 108 may be provided to an analog-to-digital converter (not shown) via an amplifier (not shown).

According to a slice thickness or the number of slices, only some of a plurality of pieces of data collected by the X-ray detector 108 may be provided to an image processor 126 via the data transmitter 120, or the image processor 126 may select only some of the plurality of pieces of data.

Such a digital signal may be provided to the image processor 126 via the data transmitter 120. The digital signal may be transmitted to the image processor 126 by wire or wirelessly.

A sensor 110 may collect various data about the outside of the mobile CT imaging apparatus 100.

Particularly, the sensor 110 may collect various position data that may occur while the mobile CT imaging apparatus 100 is moved for the CT imaging, and may detect an obstacle located in an expected path for performing the CT imaging.

As mentioned above, without fixing the table T, the mobile CT imaging apparatus 100 may image the object Ob while being moved to the Z axis direction. Accordingly, the gantry 102 moved by the mover 140 may be shaken because a space, in which the mobile CT imaging apparatus 100 performs the CT imaging, is various. The disclosed sensor 110 may collect position data to identify shaking of the gantry 102 and then applies the vibration to the image processing.

The position data measured by the sensor 110 may include a variety of data such as a movement value measured when the main body 101 is moved, a rotation angle measured when a moving direction of the main body 101 is changed due to an uneven floor or an obstacle, or a vibration value when the gantry 102 is rotated by the rotation driver 105.

The sensor 110 may include various hardware sensors capable of collecting position data, such as an ultrasonic sensor, a laser sensor, an acceleration sensor, or a gyro sensor, and may include various components such as a beam projector and a camera for detecting an obstacle. In addition, as for each sensor provided in hardware, a plurality of sensors not a single sensor may be provided in the main body 101. Detailed description thereof will be described later with reference to other drawings.

Meanwhile, the position data and the obstacle detected by the sensor 110 are transmitted to a controller 118, and the operation of the image processor 126 and the mover 140 is determined by the controller 118.

The controller 165 may be a component for controlling the overall of the mobile CT imaging apparatus 100 and may be implemented using a memory (not shown) storing an algorithm for controlling an operation of module contained in FIG. 2 and data related to programs implementing the algorithm, and a processor (not shown) performing the above mentioned operation using the data stored in the memory. The memory and the processor may be implemented in separate chips, or a single chip.

A storage 124 may store data obtained by the DAS 116 and data measured by the sensor 110. In addition, the storage 124 may store various data such as image data in which image processing is performed, to be described later, and an image generated based on the image data.

The storage 124 may include at least one storage medium from among a flash memory-type storage medium, a hard disk-type storage medium, a multimedia card micro-type storage medium, card-type memories (e.g., an SD card, an XD memory, and the like), random access memory (RAM), static random access memory (SRAM), read-only memory (ROM), electrically erasable programmable ROM (EEPROM), programmable ROM (PROM), magnetic memory, a magnetic disc, and an optical disc.

The image processor 126 may receive data acquired by the DAS 116 (e.g., pure data that is data before processing), via the data transmitter 120, and may perform pre-processing. The image processor 126 may generate a CT image using image data that is generated through the pre-processing.

For example, the pre-processing may include a process of correcting a sensitivity irregularity between channels, and a process of correcting signal loss due to a rapid decrease in signal strength or due to the presence of an X-ray absorbing material such as metal.

Image data on which the pre-processing is performed by the image processor 126 may be referred to as raw data or projection data. The projection data may a group of data values that correspond to the intensity of the X-ray that has passed through the object 10. For convenience of description, a group of a plurality of pieces of projection data that are simultaneously obtained from all channels at the same imaging angle is referred to as a projection data set.

The image processor 126 may generate a primary cross-sectional image by using the obtained projection data set, and generate a secondary cross-sectional image of the object by reconstructing the primary cross-sectional image. The secondary cross-sectional image may be a 3D image. In other words, the image processor 126 may reconstruct a 3D image of the object 10 by using a cone beam reconstruction method, based on the acquired projection data set.

According to an embodiment, the image processor 126 corrects the generated image data by applying the position data detected by the sensor 110 to the generated image data, and generates an X-ray computed tomography (CT) image by reconstructing the corrected image data. For example, the image processor 126 may apply the position data detected by the sensor 110 so as to perform the pre-processing. In another example, the image processor 126 may apply the position data in the process of reconstructing the 3D image. Detailed description thereof will be described later with reference to other drawings.

The image processor 126 may be implemented by a memory for storing a data related to an algorithm for converting digital data into an image or for image processing or programs implemented by algorithms, and a graphic processing unit (GPU) for performing the above mentioned operation by using the data stored the memory. In this case, the memory and the graphic processor may be implemented as separate chips. Alternatively, the memory and the graphics processor may be implemented on a single chip.

An inputter 128 may receive a variety of user input commands related to an X-ray CT imaging condition, and an image processing condition. For example, the X-ray CT imaging condition may include a plurality of tube voltages, a plurality of X-rays energy value setting, a selection of an imaging protocol, a selection of an image reconstruction method, a setting of a FOV area, the number of slices, a slice thickness, and a parameter setting about image post-processing, which are needed for the general CT imaging, as well as an operation start condition of the mobile CT imaging apparatus 100 and a movement condition of the mover 140.

The inputter 128 according to an embodiment may receive an input command regarding obstacle avoidance associated with the operation condition of the mover 140. Particularly, when an input command regarding obstacle avoidance is received, the controller 118 may control the mover 140 to maintain the straightness of the main body 101 even when the sensor 110 detects the obstacle. Detailed description thereof will be described later with reference to other drawings.

The inputter 128 may include a device for receiving a predetermined input from an external source. For example, the inputter 128 may include hardware devices such as various buttons, switches, pedals, keyboards, mice, trackballs, various levers, handles or sticks.

A display 130 may display the X-ray CT image, which is reconstructed by the image processor 126, and a variety of interfaces. For example, the display 130 may display a monochrome radiation image generated by the image processor 126.

Meanwhile, when the display 130 is implemented as a touch screen panel (TSP), the display 130 may form a mutual layer structure with the inputter 128. In this case, the inputter 128 may include graphical user interface (GUI) such as a touch pad that is software device.

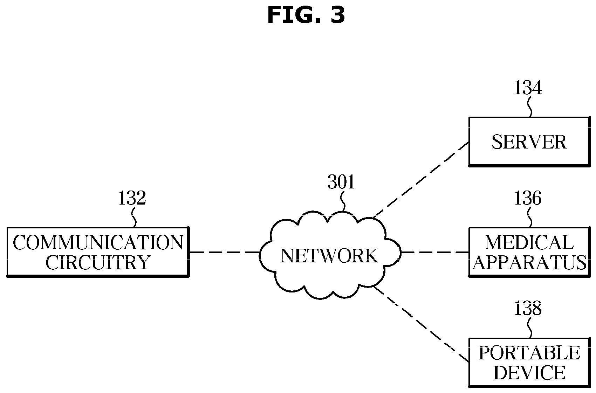

A communication circuitry 132 may perform communication with an external device and an external medical apparatus via a server 134. A description thereof will now be described with reference to FIG. 3.

The mover 140 moves the main body 101 of the mobile CT imaging apparatus 100.

Particularly, the mover 140 may include a plurality of motors 145 configured to provide a driving force to the plurality of wheels 141. As for the disclosed mobile CT imaging apparatus 100, the plurality of wheels 141 may be divided into a first wheel 141 configured to provide a rotational force to allow a user to freely move the main body through the handle, and a second wheel used for the CT imaging. That is, the mobile CT imaging apparatus 100 may be moved to the object Ob positioned on the table T, by using the first wheel, and start the CT imaging by using the second wheel. For this, the first wheel 141 may be provided in a chain shape to support the main body 101 and have a large radius of rotation, and the second wheel may be configured to be moved for precise imaging in mm units.

An object moved by the mover 140 is the main body 101 in which the configuration of the mobile CT imaging apparatus 100 is provided. However, for convenience of description, it will be assumed that the mover 140 moves the gantry 102.

FIG. 3 is a diagram illustrating a configuration of a communication circuitry.

The communication circuitry 132 may be connected to a network 301 by wire or wirelessly, and thus may perform communication with the server 134, an external medical apparatus 136, or an external device 138. The communication circuitry 132 may exchange data with a hospital server or other medical apparatuses in a hospital connected via a picture archiving and communication system (PACS).

In addition, the communication circuitry 132 may perform data communication with the external device 138, according to the digital imaging and communications in medicine (DICOM) standard.

The communication circuitry 132 may transmit and receive data related to diagnosing the object 10, via the network 301. Further, the communication circuitry 132 may transmit and receive a medical image obtained from the other medical apparatus 136 such as a magnetic resonance imaging (MRI) apparatus, or an X-ray apparatus.

Further, the communication circuitry 132 may receive a diagnosis history or a medical treatment schedule about a patient from the server 134 and may use the diagnosis history or the medical treatment schedule to diagnose the patient. In addition, the communication circuitry 132 may perform data communication not only with the server 134 or the medical apparatus 136 in a hospital but also with the portable device 138 of a user or patient.

The communication circuitry 132 may transmit information about a device error, or information about a quality control status to a system manager or a service manager via the network 301, and may receive a feedback regarding the information from the system manager or service manager.

At least one component may be added or deleted to correspond to the performance of the components of the mobile CT imaging apparatus 100 illustrated in FIGS. 1 to 3. In addition, the mutual position of the components may be changed in accordance with the performance or structure of the system. Some of the components shown in FIGS. 1 to 3 may be software components and/or hardware components such as Field Programmable Gate Arrays (FPGAs) and Application Specific Integrated Circuits (ASICs).

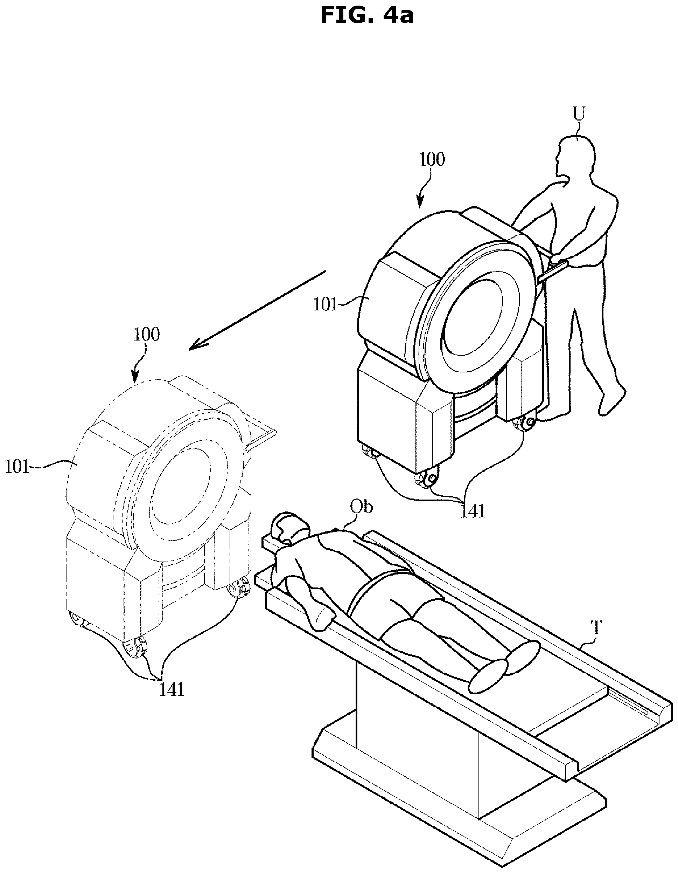

FIGS. 4A to 4C are views illustrating a mover of the mobile CT imaging apparatus. In order to avoid duplicates, the description thereof will be described together.

Referring to FIG. 4A, by using the first wheel 141, a user U moves the mobile CT imaging apparatus 100 according to an embodiment to near the object Ob. Particularly, through the first wheel 141, the user U may move the mobile CT imaging apparatus 100 to a position corresponding to an anatomical position of the object Ob to be imaged.

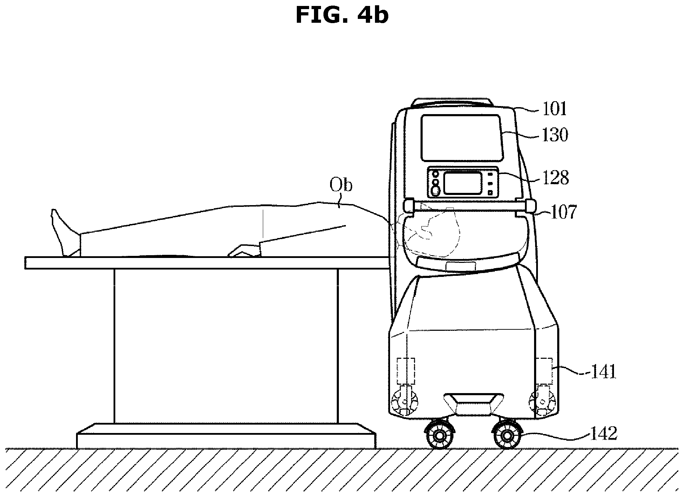

For example, the user U may position the mobile CT imaging apparatus 100 to the position of FIG. 4B to image the head of the object Ob. When it is identified that the mobile CT imaging apparatus 100 is moved to a position suitable for the CT imaging, the user U may replace the first wheel 141 with the second wheel 142.

The mobile CT imaging apparatus 100 may replace the first wheel 141 with the second wheel 142 through the inputter 128. After the second wheel 142 completely protrudes from the inside of the main body 101, the first wheel 141 may be inserted into the main body 101.

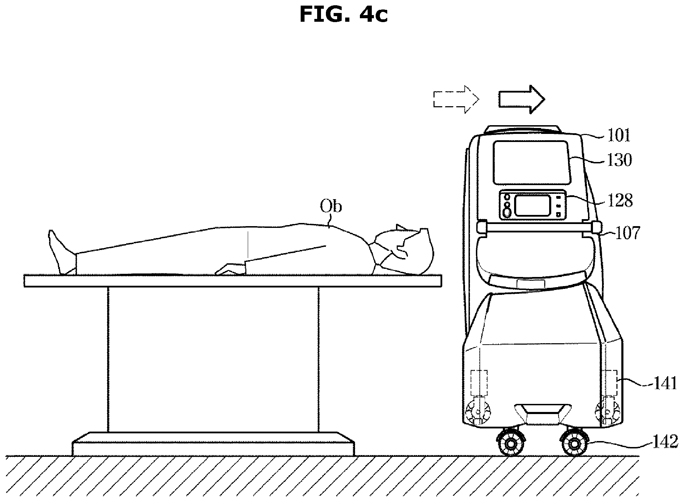

Referring to FIG. 4C, the inputter 128 may receive an input command relating to the CT imaging start from the user U. The mobile CT imaging apparatus 100 rotates the gantry 102 through the rotation driver 105. In addition, the mobile CT imaging apparatus 100 is moved straight through the second wheel 142 in the Z-axis direction, particularly, in a direction away from the object Ob.

When the mobile CT imaging apparatus 100 is moved while rotating the gantry 102, the sensor 110 detects the position data, and the controller 118 performs image processing using the position data detected during the image processing.

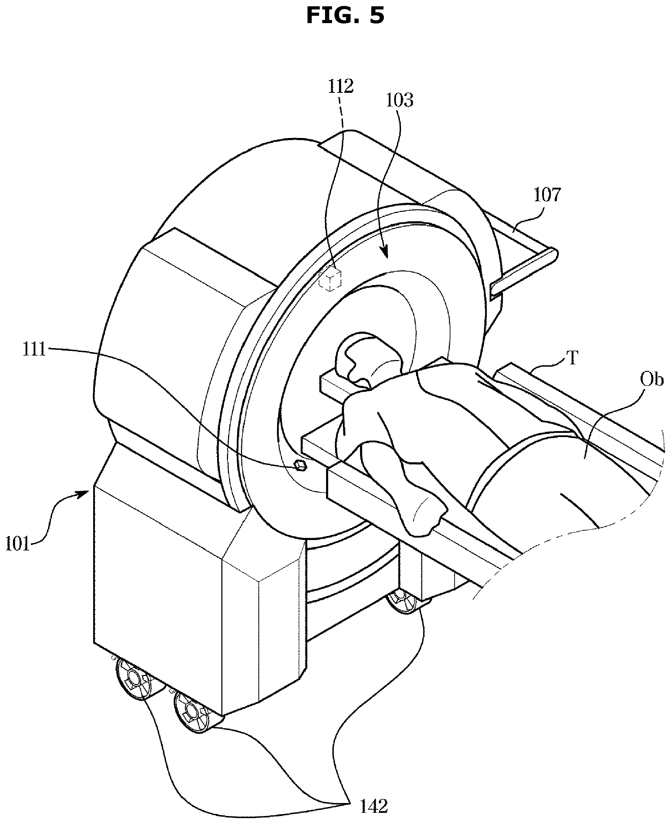

FIG. 5 is a view of the sensor 110 according an embodiment of the disclosure.

As mentioned above, the sensor 110 may correspond to a hardware device configured to detect position data, and may include at least one of an ultrasonic sensor, a laser sensor, an acceleration sensor, or a gyro sensor. The sensor 110 may be provided in a cover 103 of the gantry 102 as shown in FIG. 5.

Referring to FIG. 5, the sensor 110 according to an embodiment may include a distance sensor 111 arranged at a lower end of the cover 103 with respect to the rotation axis RA and configured to measure a distance from the table T that is to be inserted to the gantry 102.

For example, the distance sensor 111 may include an ultrasonic sensor or a laser sensor. A plurality of distance sensors 111 may be provided to detect a distance from the lower end of the cover 103 to an end of the table T.

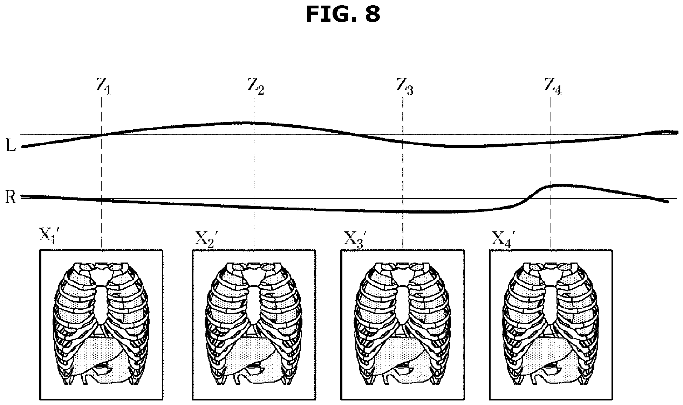

The controller 118 calculates a movement value according to the movement of the gantry 102 and a rotation angle according to the movement of the gantry 102 based on the respective position data measured by the plurality of distance sensors 111. An embodiment of the position data that the controller 118 calculates through the distance sensor 111 will be described in detail later with reference to FIG. 8.

According to another embodiment, the sensor 110 may include a gyro sensor 112 provided at an upper end of the cover 103 with respect to the rotation axis RA, and configured to detect a position of the gantry 102, which is straightly moved at the start of the CT imaging, and a shake of the gantry 102 caused by the rotation of the gantry 102. A single gyro sensor 112 may be provided, and by using the detection value of the gyro sensor 112, the controller 118 may calculate a movement value, and a rotation angle and a vibration value caused by the rotation of the gantry.

The gyro sensor 112 may be replaced with an acceleration sensor, and may further include various sensors configured to measure position data in addition to the various sensors described above.

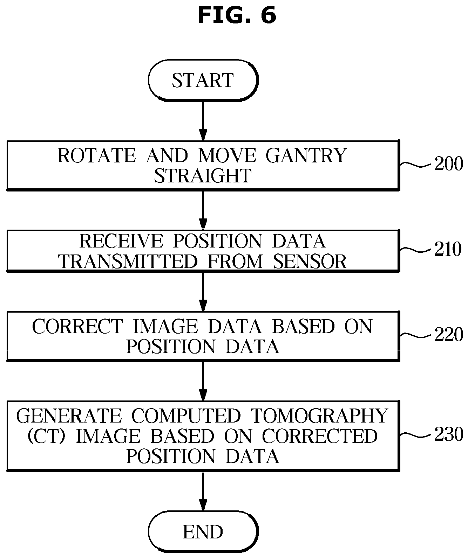

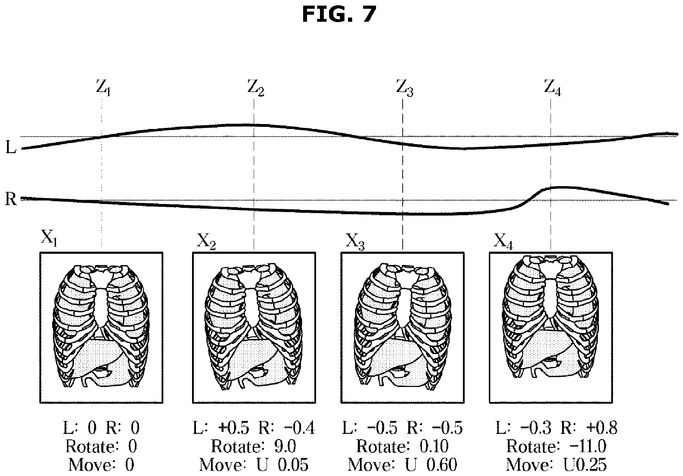

FIG. 6 is a flowchart illustrating a control method of a medical imaging apparatus according to an embodiment of the disclosure, FIG. 7 is a view illustrating an example of receiving position data from the sensor, and FIG. 8 is a view illustrating a method of correcting image data based on the position data.

Referring to FIG. 6, the controller 118 rotates the gantry 102 while moving straight the gantry 102 forward (200).

Particularly, while emitting X-rays, the controller 118 rotates the gantry 102 through the rotation driver 105 and moves the gantry 102 away from the object Ob through the mover 140. While the gantry 102 is moved, the sensor 110 collects position data.

The controller 118 receives the position data transmitted from the sensor 110 (210).

The position data transmitted by the sensor 110 may vary. Particularly, when the embodiment of the sensor 110 includes both the distance sensor 111 and the gyro sensor 112, the controller 118 may store the position data in the storage 124 as shown in FIG. 7.

Referring to FIG. 7, the controller 118 may match the position data collected in sections Z1 to Z4, in which the gantry is moved, with the image data generated in each section.

Particularly, the plurality of distance sensors 111 may measure a distance L from a left end of the table T and a distance R from a right end of the table T, and the gyro sensor 112 may measure a rotation angle (Rotate) or a vibration value (Move: U)

The measured position data is transmitted to the controller 118, and the controller 118 compares a point of time, at which the position data is collected, with the moving section of the gantry 102.

For example, in a section Z1, the gantry 102 may image the chest of the object Ob while being ideally moved straight. In the section Z1, the controller 118 may receive the position data indicating that a distance from the left side of the table is 0 (zero) and a distance from the right side of the table is 0 (zero), the rotation does not occur and the vibration hardly occurs in comparison with a reference. The controller 118 may match the received plurality of pieces of position data with image data X1.

The right side of the gantry 102 may be raised by an obstacle located on a straight path of the right wheel of the second wheel 142 in a section Z2. When all four wheels contained in the second wheel 142 are rotated at the same rotation speed, the gantry 102 may be rotated clockwise by the obstacle located on the right wheel. The position data collected in such a situation may indicate that the distance from the left side of the table is increased by 0.5, the distance from the right side of the table is reduced by 0.4, a path is rotated at 9 degrees with respect to the straight path and a vibration of U 0.05 occurs. Therefore, image data X2 generated in the section Z2 may be more deviated than the image data X1 in the section Z1.

In a section Z3, the gantry 102 may be moved straight on an uphill region. In this case, the collected position data may indicate that the distance from the left side of the table is reduced by 0.5, the distance from the right side of the table is reduced by 0.5, a path is rotated at 0.1 degrees with respect to the straight path and a vibration of U 0.60 occurs. The controller 118 may match the position data measured in the section Z3 with image data X3 generated later than the image data X1 generated in the section Z1.

In a section Z4, the left side of the gantry 102 may be raised by an obstacle located on the left side on the straight path which is opposite to the situation of the section Z2. The position data collected in this situation may indicate that the distance from the left side of the table is reduced by 0.3, the distance from the right side of the table is increased by 0.8, a path is rotated counterclockwise by 11 degrees with respect to the straight path, and a vibration of U 0.25 occurs. The controller 118 may match the position data measured in the section Z4 with image data X4 rotated to the right side than the image data X1 generated in the section Z1.

Referring again to FIG. 6, the controller 118 corrects image data based on the matched position data (220).

The controller 118 according to an embodiment corrects the image data by applying the position data matched to the generated image data X1 to X4. As a result of the correction, the image data shown in FIG. 7 becomes image data XI' to X4' that are reconstructed by applying the position data of the sensor 110 as illustrated in FIG. 8.

The controller 118 generates a CT image based on the corrected image data (230).

For example, the controller 118 may generate a 3D image of the chest by reconstructing the corrected image data XI' to X4' as illustrated in FIG. 8. When the 3D image is generated based on the image data X1 to X4, in which the correction using the position data is not performed, the 3D image may include an error caused by the respective image data distorted by the movement. The controller 118 corrects the image data before generating the CT image, and thus it is possible to provide the restraint on a change in the surrounding environment, and prevent a misdiagnosis or a medical accident due to an incorrect CT image.

Meanwhile, the disclosed embodiment may be applied to not only the CT imaging in which the gantry 102 is moved and rotated. That is, the correction of the image data by the position data may be applied to CT imaging in which an object Ob is scanned without the rotation of the gantry 102.

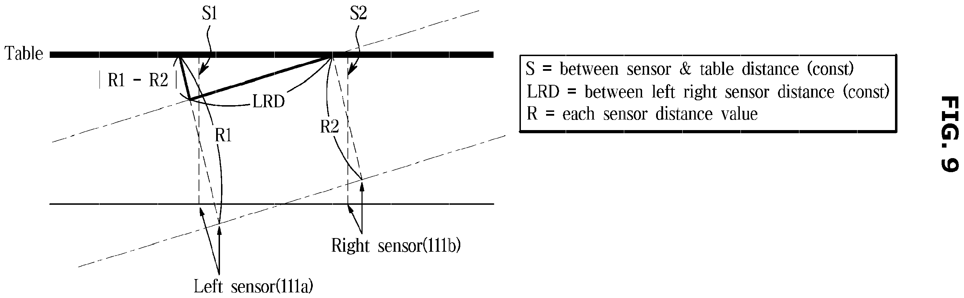

FIG. 9 is a view illustrating an example of the position data.

FIGS. 6 to 8 illustrate the embodiment in which the image date is corrected using the plurality of pieces of position data collected by the sensor 110 including the plurality of sensors such as the distance sensor 111 and the gyro sensor 112.

However, in the disclosed embodiment, the sensor 110 may include only the distance sensor 111. The plurality of distance sensor 111 may be ultrasonic sensors 111a and 111b arranged in the lower end of the cover 103 of the gantry 102. The controller 118 may measure a relative distance of each end of the table T through the two ultrasonic sensors 111a and 111b. The controller 118 may calculate a movement value of the gantry 102 and a rotation angle of the gantry 102 based on measurement distance values of the ultrasonic sensors 111a and 111b through the following calculation method.

Referring to FIG. 9, the distance sensor 111 may measure distances S1 and S2 as a reference to the table T. S1 and S2 represent detection values of the distance sensor 111 when the gantry 102 is moved straight along the flat floor without the obstacle. S1 and S2 may be stored in the storage 124. R1 represents the distance between the left sensor 111a of the distance sensor 111 and the table T. R2 represents the distance between the right sensor 111b of the distance sensor 111 and the table T. In addition, the left right senor distance (LRD) is a distance value of each of the plurality of distance sensors 111, which is also determined at the time when the sensor is provided.

When a moving direction is changed by the obstacle while the gantry 102 is moved by the mover 140, as illustrated in FIG. 9, a distance R1 between the left sensor 111a of the distance sensor 111 and the table T may be increased.

Based on the detection value of the sensor, the controller 118 may calculate a rotation angle $\theta$ caused by the obstacle, as shown in equation 1 below.

[Equation 1]

$$\theta = \tan\text{-}1\,(|\,R1\text{-}R2\,|\,/\,LRD)$$

In addition, the gantry 102 may emit X-rays on an inclined floor such as the uphill or the downhill. In this case, the controller 118 may calculate a movement value in which the gantry 102 is moved, using equation 2 below.

[Equation 2]

$$R1\text{-}S1\text{-}|\,R1\text{-}R2\,|$$

When a result value calculated by equation 2 is 0 (zero), it may be determined that the gantry 102 images the object Ob while being moved on the flat floor.

Meanwhile, the position data described with reference to FIG. 9 is merely an example of position data acquired by the distance sensor 111. That is, the disclosed mobile CT imaging apparatus 100 may calculate the movement value or the rotation angle from the distance sensor 111 through various methods other than those described above, and the CT imaging apparatus 100 may calculate the movement value or the rotation angle through sensors other than the distance sensor 111.

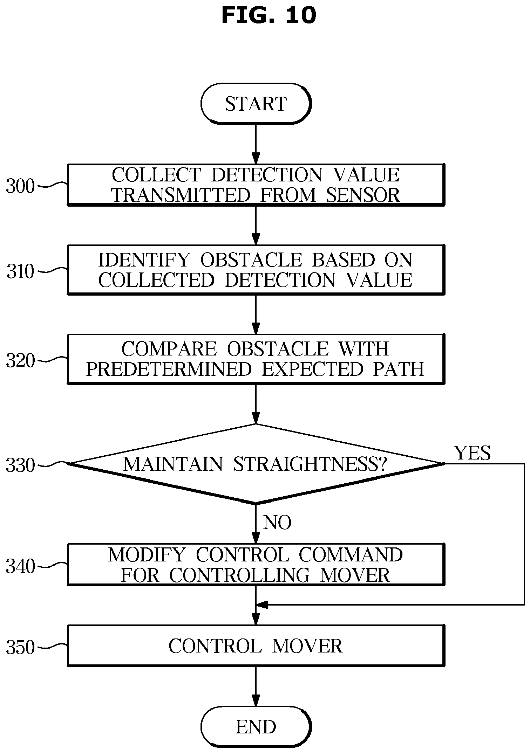

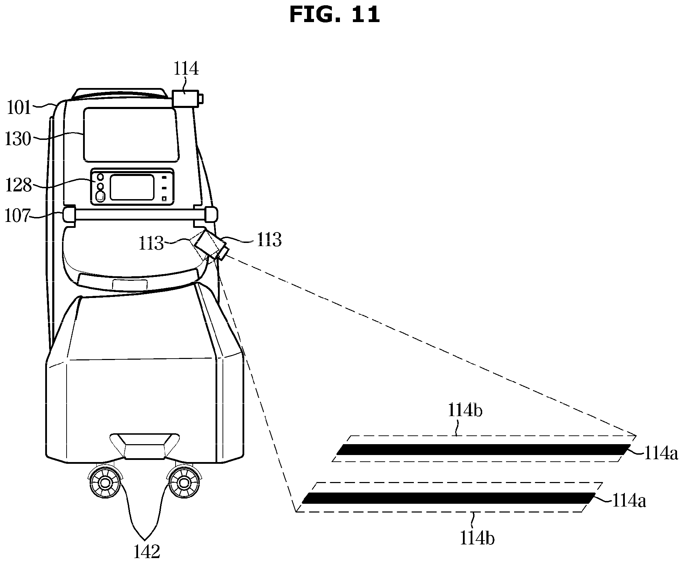

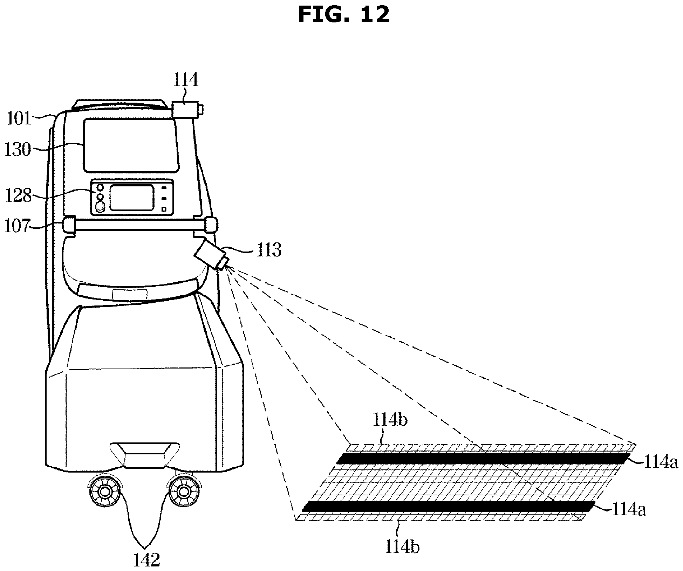

FIG. 10 is a flowchart illustrating a control method of a mover according to another embodiment of the disclosure. FIGS. 11 and 12 are views illustrating an example of identifying an obstacle. In order to avoid duplicates, the description will be described together.

The controller 118 receives the detection value measured by the sensor 110 (300).

Referring to FIGS. 11 and 12, a sensor 110 according to another embodiment may further include a beam projector 113 configured to emit laser beams to a direction in which the gantry 102 is moved, and a camera 114 configured to image an obstacle distinguished by the laser beams emitted by the beam projector 113. The controller 118 may receive a detection value of the sensor 110 according to another embodiment, that is, an image imaged by the camera 114.

The controller 118 identifies the obstacle based on the received detection value (310).

The obstacle may include all of various things that may interfere with performing the straight movement of the gantry 102. For example, the obstacle may include a groove into which the second wheel 142 may be inserted, in addition to the object interfering with the path of the second wheel 142.

The controller 118 compares the obstacle with a predetermined expected path.

Referring to FIG. 11, the controller 118 may distinguish the predetermined image path 114 from a floor image 114a imaged with the one-dimensional laser beam emitted by the beam projector 113. The predetermined path 114b may correspond to a path that is set based on the straight movement of the second wheel 142.

Referring to FIG. 12, according to another embodiment, the beam projector 113 may emit laser beams in a wide range, unlike in FIG. 11, and the camera 114 may transmit a two-dimensional grid image 114a to the controller 118. The controller 118 may distinguish the expected path 114b from the gird image 114a and identify whether or not an obstacle is placed on the expected path.

Meanwhile, the range in which the beam projector 113 emits laser beams may vary according to a protocol in which imaging is performed, as illustrated in FIGS. 11 and 12. For example, when scanning the entire table T, the controller 118 may control the beam projector 113 to emit laser beams according to the length of the table T.

On the contrary, when the imaging protocol is to image only the head of the object Ob, the controller 118 may limit the range of the emission to the predetermined range in which the beam projector 113 emits laser beams.

Referring again to FIG. 10, the controller 118 may determine whether to maintain the straightness based on the comparison result (330).

When the obstacle is not detected or when it is determined that the obstacle does not effect on the straightness although the obstacle is detected, the controller 118 operates the mover 140 and performs the imaging without changing the control command of the mover 140.

However, when the obstacle is detected, the controller 118 modifies the control command for controlling the mover 140 (340).

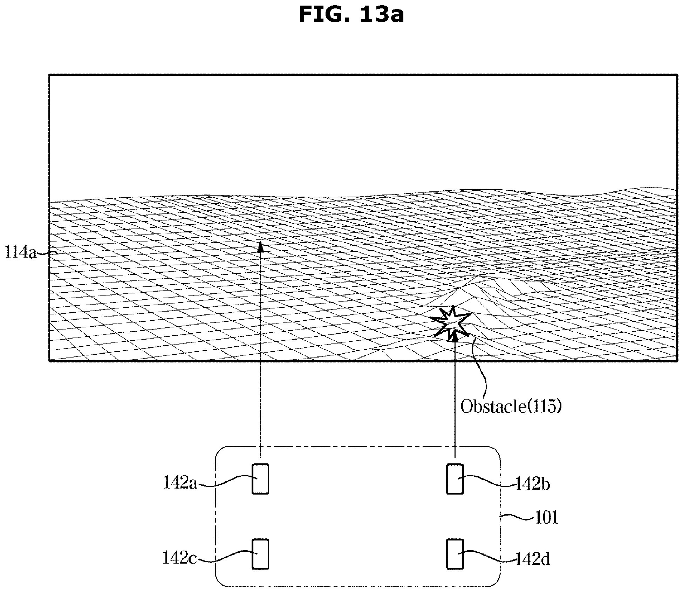

Particularly, a method of modifying the control command may vary, but in an example, the controller 118 may modify the control command so that the gantry 102 may have an effect such as moving straight despite of the presence of the obstacle. Detailed description thereof will be described later with reference to FIGS. 13A to 13C.

The controller 118 controls the mover 140 according to the modification result (350).

FIGS. 13A to 13C are views illustrating an example of controlling the mover based on an obstacle identified. In order to avoid duplicates, the description will be described together.

Referring to FIG. 13A, the camera 114 may image the two-dimensional grid image 114a generated by the beam projector 113.

In the grid image 114a, the controller 118 may identify whether or not an obstacle 115 is present in the predetermined expected path. Particularly, the controller 118 may determine that the second wheels 142b and 142d provided on the right side of the four second wheels 142a to 142d are moved to the obstacle along the expected path.

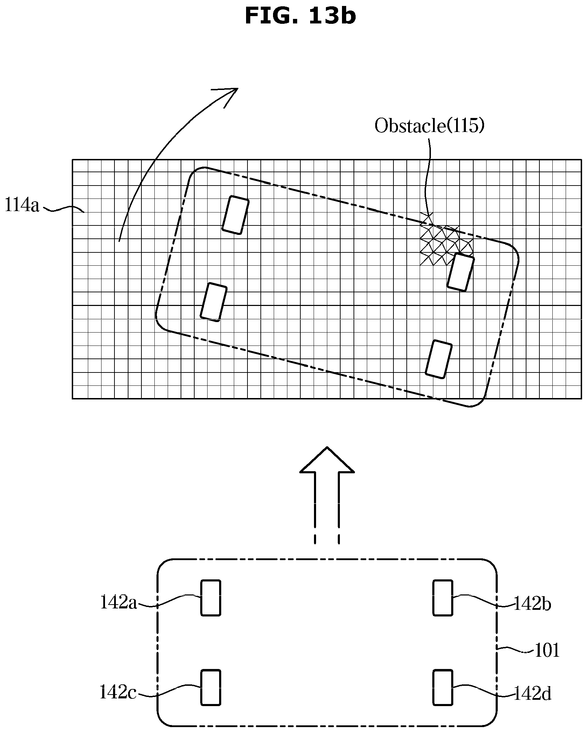

Referring to FIG. 13B, the controller 118 may rotate the four second wheels 142a to 142d at the same rotation speed based on the control command that is not changed.

In the case of the obstacle such as FIG. 13A, the path, on which the second wheel 142b on the right side is moved, is longer than the path, on which the second wheel 142a on the left side is moved. Therefore, when the control command is not changed, the main body 101 is rotated clockwise.

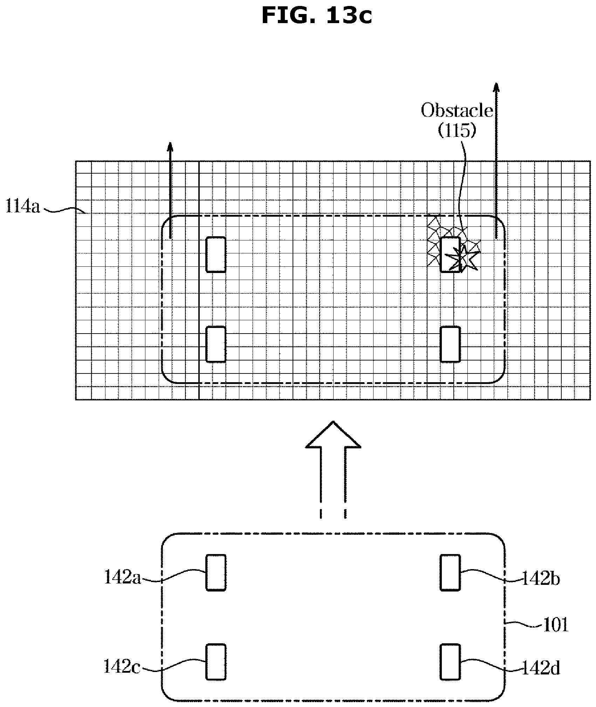

In order to prevent this, when the wheels are moved to the obstacle, the controller 118 may allow the motor, which is configured to drive the second wheels 142b and 142d on the right side, to have greater driving force. Referring to FIG. 13C, the controller 118 may provide the same rotational force to the three second wheels 142a, 142c, and 142d, and provide a high driving force to the second wheel 142b positioned at the obstacle, thereby maintain the straightness of the main body 101.

However, FIGS. 13A to 13C are merely an example and thus the embodiment may include a variety of control methods.

As is apparent from the above description, the mobile computed tomography (CT) imaging apparatus and the control method of the same may provide the usability of the mobile CT imaging apparatus and the restraint on a change in the surrounding environment by applying a vibration, which may occur in computed tomography imaging of the mobile CT imaging apparatus, to an image reconstruction.

The mobile CT imaging apparatus and the control method of the same may provide a CT image, which is improved than a CT image acquired by the conventional mobile CT imaging apparatus, and prevent misdiagnosis caused by an incorrect CT image.

Although a few embodiments of the disclosure have been shown and described, it would be appreciated by those skilled in the art that changes may be made in these embodiments without departing from the principles and spirit of the disclosure, the scope of which is defined in the claims and their equivalents.

Description of symbols

| | | | |
|---|---|---|---|
| 100: | mobile computed tomography imaging apparatus | | |
| 101: | main body | | |
| 102: | gantry | 103: | cover |
| 104: | rotating frame | 105: | rotation driver |
| 110: | sensor | 118: | controller |

**Claims**

**1.** A medical imaging apparatus comprising a rotatable gantry in which an X-ray generator configured to generate X-rays and emit the X-rays to an object and an X-ray detector configured to detect the X-rays emitted from the X-ray generator are arranged to face each other, the medical imaging apparatus comprising:

a mover configured to move the medical imaging apparatus;
a sensor configured to measure position data according to the movement of the gantry; and
a controller configured to generate image data based on the detected X-rays, configured to correct the image data based on the measured position data, and configured to generate an X-ray computed tomography (CT) image based on the corrected image data.

2. The medical imaging apparatus of claim 1, wherein
the position data comprises at least one of a movement value according to the movement of the gantry, a rotation angle according to the movement of the gantry, or a vibration value according to the rotation of the gantry, wherein the controller corrects the image data based on a plurality of pieces of position data.

3. The medical imaging apparatus of claim 1, wherein
the controller generates the image data based on the detected X-rays and the position data.

4. The medical imaging apparatus of claim 1, wherein
the controller matches position data that is detected by the sensor in each section in which the gantry is moved, with image data that is generated according to the movement of the gantry, and the controller corrects the image data based on the matched position data.

5. The medical imaging apparatus of claim 1, wherein
the sensor comprises at least one of an ultrasonic sensor, a laser sensor, an acceleration sensor, or a gyro sensor.

6. The medical imaging apparatus of claim 1, wherein
the sensor comprises a plurality of sensors configured to measure a distance to a table on which the object is placed.

7. The medical imaging apparatus of claim 5, further comprising:

a storage configured to store first position data comprising distances between the table and the plurality of sensors,
wherein the controller compares second position data detected according to the movement of the gantry with the first position data, and corrects the image data based on a comparison result.

8. The medical imaging apparatus of claim 1, wherein
the sensor detects an obstacle positioned in a direction in which the gantry is moved,
wherein the controller controls the mover based on a predetermined expected path and the detected obstacle.

9. The medical imaging apparatus of claim 8, wherein
the sensor comprises
a beam projector configured to emit laser beams to the direction in which the gantry is moved; and a camera configured to image an obstacle distinguished by the laser beam,
wherein the controller detects an obstacle based on the image imaged by the camera.

10. The medical imaging apparatus of claim 9, wherein
the controller controls the mover to allow the gantry to move straight on the detected obstacle.

11. The medical imaging apparatus of claim 9, wherein
the controller controls the beam projector to emit the laser beam based on a length of the table on which the object is placed.

12. The medical imaging apparatus of claim 7, wherein
the mover comprises a first wheel, a first motor configured to provide a driving force to the first wheel, a second wheel configured to move the gantry during the X-rays are emitted, and a second motor configured to provide a driving force to the second wheel,
wherein the controller controls the second motor to regulate a rotation speed of the second motor based on the detected obstacle.

13. A control method of a medical imaging apparatus comprising a rotatable gantry in which an X-ray generator configured to generate X-rays and emit the X-rays to an object and an X-ray detector configured to detect the X-rays emitted from the X-ray generator are arranged to face each other, the control method comprising:

moving the medical imaging apparatus;
generating image data based on the detected X-rays;
measuring position data according to movement of the gantry;
correcting the image data based on the measured position data; and
generating an X-ray computed tomography (CT) image based on the corrected image data.

14. A control method of a medical imaging apparatus comprising a rotatable gantry in which an X-ray generator configured to generate X-rays and emit the X-rays to an object and an X-ray detector configured to detect the X-rays emitted from the X-ray generator are arranged to face each other, the control method comprising:

moving the medical imaging apparatus;
measuring position data according to movement of the gantry;
generating the image data based on the measured position data; and
generating an X-ray computed tomography (CT) image based on the generated image data.

15. The control method of claim 13, wherein
the position data comprises at least one of a movement value according to the movement of the gantry, a rotation angle according to the movement of the gantry, or a vibration value according to the rotation of the gantry,
wherein the correction of the image data comprises correcting the image data based on a plurality of pieces of position data.

# FIG. 1

FIG. 2

EP 3 689 243 A1

100    107

102
104    112
118
106
CONTROLLER
STORAGE    124
IMAGE PROCESSOR    126
ROTATION DRIVER
105
INPUTTER    128
FOV
DISPLAY    130
RA
SENSOR    110
114
108    X-RAY DETECTOR
116    DATA ACQUISITION SYSTEM (DAS)
132
COMMUNICATION CIRCUITRY
120    DATA TRANSMITTER
SERVER (134)
MOVER    140

15

# FIG. 3

# FIG. 4a

# FIG. 4b

# FIG. 4c

# FIG. 5

# FIG. 6

```
┌─────────────┐
│    START    │
└─────────────┘
       │
       ▼
┌─────────────────────────────┐
│   ROTATE AND MOVE GANTRY     │ ～ 200
│          STRAIGHT            │
└─────────────────────────────┘
       │
       ▼
┌─────────────────────────────┐
│     RECEIVE POSITION DATA    │ ～ 210
│   TRANSMITTED FROM SENSOR    │
└─────────────────────────────┘
       │
       ▼
┌─────────────────────────────┐
│  CORRECT IMAGE DATA BASED ON │ ～ 220
│        POSITION DATA         │
└─────────────────────────────┘
       │
       ▼
┌─────────────────────────────┐
│  GENERATE COMPUTED TOMOGRAPHY│ ～ 230
│  (CT) IMAGE BASED ON CORRECTED│
│        POSITION DATA         │
└─────────────────────────────┘
       │
       ▼
┌─────────────┐
│     END     │
└─────────────┘
```

# FIG. 7

L: 0 R: 0
Rotate: 0
Move: 0

L: +0.5 R: −0.4
Rotate: 9.0
Move: U 0.05

L: −0.5 R: −0.5
Rotate: 0.10
Move: U 0.60

L: −0.3 R: +0.8
Rotate: −11.0
Move: U0.25

# FIG. 8

# FIG. 9

S = between sensor & table distance (const)
LRD = between left right sensor distance (const)
R = each sensor distance value

Table

S1

S2

| R1 − R2 |

LRD

R1

R2

Left sensor(111a)

Right sensor(111b)

# FIG. 10

# FIG. 11

# FIG. 12

## FIG. 13a

114a

Obstacle(115)

142a 142b

101

142c 142d

# FIG. 13b

Obstacle(115)

114a

142a
142b
101
142c
142d

# FIG. 13c

Obstacle (115)

114a

142a
142b
101
142c
142d

Europäisches
Patentamt

European
Patent Office

Office européen
des brevets

# EUROPEAN SEARCH REPORT

Application Number

EP 20 15 4978

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/243151 A1 (SHIH ANG [US]) 19 September 2013 (2013-09-19) | 1-5, 13-15 | INV. A61B6/10 A61B6/00 |
| A | * paragraph [0031] - paragraph [0062] * ----- | 6-12 | |
| A | US 2018/110494 A1 (HSIEH JONG-JYH [KR]) 26 April 2018 (2018-04-26) * paragraph [0055] - paragraph [0215] * ----- | 1-15 | ADD. A61B6/03 |
| A | US 2018/242935 A1 (BOUVIER BERNARD [FR] ET AL) 30 August 2018 (2018-08-30) * paragraph [0040] - paragraph [0143] * ----- | 1-15 | |

TECHNICAL FIELDS
SEARCHED      (IPC)

A61B

The present search report has been drawn up for all claims

| Place of search | Date of completion of the search | Examiner |
|---|---|---|
| Munich | 24 June 2020 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 15 4978

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2020

| Patent document cited in search report | Publication date | Patent family member(s) | Publication date |
|---|---|---|---|
| US 2013243151 A1 | 19-09-2013 | NONE | |
| US 2018110494 A1 | 26-04-2018 | CN 107981884 A<br>KR 101844515 B1<br>US 2018110494 A1 | 04-05-2018<br>14-05-2018<br>26-04-2018 |
| US 2018242935 A1 | 30-08-2018 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190013463